# EUROPEAN PATENT APPLICATION

(11) **EP 3 095 782 A1**
(43) Date of publication of application: **23.11.2016**
(21) Application number: 15382257.2
(22) Date of filing: 18.05.2015
(51) Int. Cl.: C07D 233/64

(54) **NEW METHOD FOR PREPARING 3-(4-METHYL-1H-IMIDAZOL-1-YL)-5-(TRIFLUOROMETHYL)BENZENAMINE**

(71) Applicant: Esteve Química, S.A., 08024 Barcelona (ES); Esteve Huayi Pharmaceutical Co. Ltd., Shaoxing, Zhejiang 312071 (CN)
(72) Inventor: Hong, Jiang, 312071 Shaoxing (CN); Hong, Wu, 312071 Shaoxing (CN); Xiaofei, Xu, 312071 Shaoxing (CN); Guorong, Zheng, 312071 Shaoxing (CN); Bartra,SANMARTÌ, Marti, E-08024 BARCELONA (ES); Lupón, Pilar, E-08024 BARCELONA (ES)
(74) Representative: ABG Patentes, S.L.

(57) **Abstract**

The present invention relates to a new method for preparing 3-(4-methyl-1H-imidazol-1-yl)-5-(trifluoromethyl)-benzenamine. The invention also relates to a new intermediate of formula (XI) used in this method and to a method for preparing nilotinib comprising the step of preparing 3-(4-methyl-1H-imidazol-1-yl)-5-(trifluoromethyl)-benzenamine by the method of the invention.

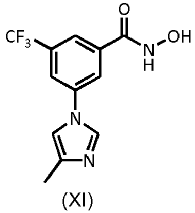

## Description

### Field of the Invention

The present invention relates to a new method for preparing 3-(4-methyl-1H-imidazol-1-yl)-5-(trifluoromethyl)-benzenamine. The invention also relates to new intermediates used in this method and to a method for preparing nilotinib comprising the step of preparing 3-(4-methyl-1H-imidazol-1-yl)-5-(trifluoromethyl)-benzenamine by the method of the invention.

### Prior Art

Nilotinib or 4-methyl-N-[3-(4-methyl-1H-imidazol-1-yl)- 5-(trifluoromethyl)phenyl]-3-[(4-pyridin-3-ylpyrimidin-2-yl) amino]benzenamine belongs to a class of tyrosine kinase inhibitors which can be used approved for the treatment of certain neoplastic diseases such as leukemia.

The chemical structure of nilotinib is represented by:

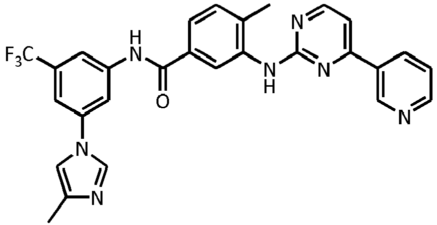

Nilotinib is marketed in most countries under the name of Tasigna (Novartis).

Nilotinib was first disclosed in patent application WO 2004/0058281 A1. The same document also describes a preparation process for the preparation of nilotinib which involves the following reaction:

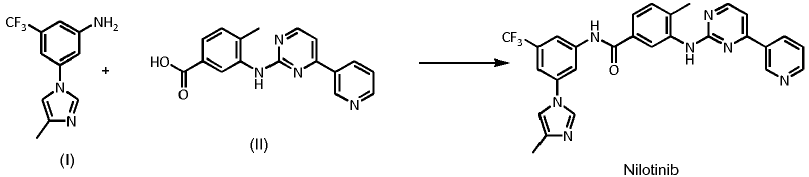

Patent application WO 2004/0058281 A1 also describes a 4 step process for the preparation of the compound of formula (I), 3-(4-methyl-1H-imidazol-1-yl)-5-(trifluoromethyl)-benze-namine involving the following steps:

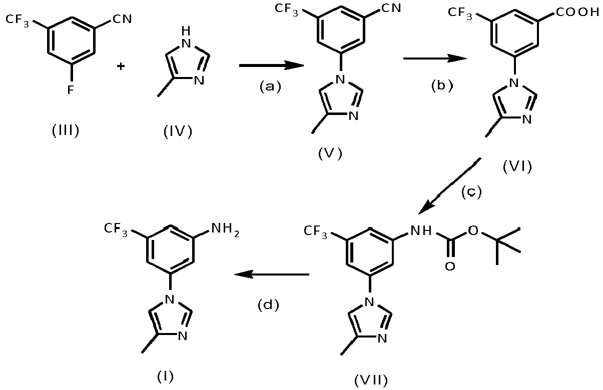

The authors of the present invention have reproduced the synthetic process described in the examples of patent application WO 2004/0058281 A1 for the preparation of compound (I), 3-(4-methyl-1H-imidazol-1-yl)-5-(trifluoromethyl)-benze-namine. When starting from compound of formula (V) the process described in said example resulted in the compound of formula (I) with an overall yield of 40,4%. Additionally the process described in WO 2004/0058281 A1 involves the use of diphenylphosphoryl azide in reaction step (c) and said reactant is known to be very toxic and a potential explosive which makes it unsuitable for industrial use.

Patent application WO 2006/135640 A2 discloses an alternative process for preparing the compound of formula (I), 3-(4-methyl-1H-imidazol-1-yl)-5-(trifluoromethyl)-benzenamine involving the following steps:

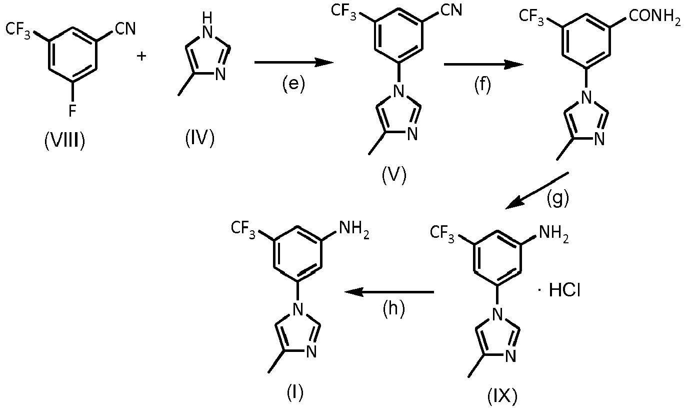

When starting from compound of formula (V) the process described in WO 2006/135640 A2 (see examples 7 and 8) is reported to result in the compound of formula (I) with an overall yield of 25.7%.

Therefore, there is a need in the state of the art for alternative methods for the synthesis of 3-(4-methyl-1H-imidazol-1-yl)-5-(trifluoromethyl)-benzenamine capable of producing the desired compound with high yields and which do not make use of reactants such as diphenylphosphoryl azide which are unsuitable of being employed at an industrial scale.

### Summary of the Invention

An object of the present invention is to provide an industrially applicable method for the synthesis of 3-(4-methyl-1H-imidazol-1-yl)-5-(trifluoromethyl)-benzenamine (I) which allows producing the compound at yields higher than those obtained when using the methods described in the art.

Another object of the present invention is to provide new intermediates for the synthesis of 3-(4-methyl-1H-imidazol-1-yl)-5-(trifluoromethyl)-benzenamine using the method of the invention.

An object of the present invention is to provide an industrially applicable method for the synthesis of nilotinib.

In one aspect of the present invention, it is provided a method for preparing 3-(4-methyl-1H-imidazol-1-yl)-5-(trifluoromethyl)-benzenamine (I) by reacting the compound of formula (XI) 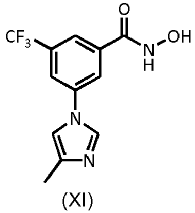 either with a base (B1) or alternatively with a dehydration agent (DA1) in a solvent (S1) to yield the compound of formula (I)

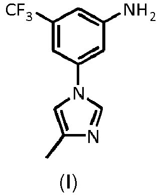

In another aspect of the invention a compound of formula (XI) is provided.

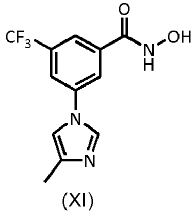

In another aspect of the invention it is provided a method for preparing Nilotinib or 4-methyl-N-[3-(4-methyl-1H-imidazol-1-yl)- 5-(trifluoromethyl)phenyl]-3-[(4-pyridin-3-ylpyrimidin-2-yl)amino)-benzenamine) comprising the following steps:
a) reacting the compound of formula (XI) 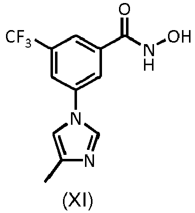 either with a base (B1) or alternatively with a dehydration agent (DA1) in a solvent (S1) to yield the compound of formula (I) 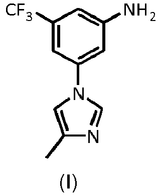
b) reacting the compound of formula (I) with a compound of formula (IIa) optionally in the presence of a dehydrating agent (DA4) and an inert base (B4) and/or suitable catalyst, and optionally in the presence of an inert solvent (S4). 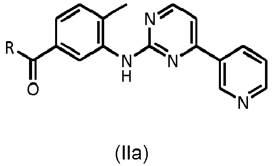 wherein the group COR is selected from the group consisting of carboxylic acids, acyl halides and carboxylic esters;
   to yield nilotinib

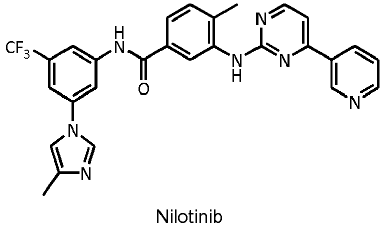

### Detailed Description of the Invention

The authors have developed an industrially applicable method for the synthesis of 3-(4-methyl-1H-imidazol-1-yl)-5-(trifluoromethyl)-benzenamine (I), by reacting a compound of formula (XI) either with a base (B1) or alternatively with a dehydration agent (DA1) in a solvent (S1) to yield the compound of formula (I). Said method constitutes a first aspect of the present invention.

In one embodiment of the invention the reaction step from (XI) to (I) is carried out in the presence of a strong base (B1) (i.e. a base having a pKb of less than 5. Particularly, useful bases are selected from the group consisting of inorganic hydroxides and carbonates such as LiOH, NaOH, KOH, CsOH, Ca(OH)₂, Zn(OH)₂, Na₂CO₃ or K₂CO₃. In a more preferred embodiment the base is a metallic carbonate such as Na₂CO₃, K₂CO₃ and ZnCO₃.

In an alternative embodiment of the invention the above mentioned reaction step is carried out in the presence of a dehydrating agent (DA1) selected from the group consisting of dicyclohexylcarbodiimide, carbonyldiimidazole, thionyl chloride, acetic anhydride, p-toluenesulfonyl chloride, cyanuric chloride, phosphorous pentoxide and N-benzyl-N-(3-dimethylaminopropyl)carbodiimide, preferably carbonyldiimidazole.

The reaction may be carried out in a solvent (S1) selected from the group consisting dimethyl sulfoxide, dimethyl formamide, ethyl acetate, dichloromethane, toluene, tetrahydrofurane, acetonitrile. When the reaction is carried out in the presence of a base the preferred solvents (S1) are selected from the group consisting of dimethyl sulfoxide and dimethyl formamide, preferably dimethyl sulfoxide. When the reaction is carried out in the presence of a dehydrating agents the preferred solvents (S1) are selected from the group consisting of ethyl acetate, dichloromethane, toluene, tetrahydrofurane and acetonitrile, preferably acetonitrile.

The compound of formula (XI) 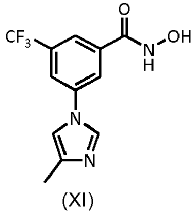 which is the starting product of the above mentioned reaction is a new compound a constitutes a second aspect of the present invention.

In another embodiment the present invention relates to a method for the synthesis of 3-(4-methyl-1H-imidazol-1-yl)-5-(trifluoromethyl)-benzenamine (I),comprising the steps:
reacting the compound of formula (X) 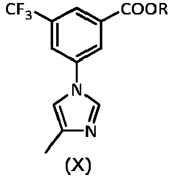 with hydroxylamine or a salt thereof in the presence of a base (B2) in a solvent (S2) to yield the compound of formula (XI) 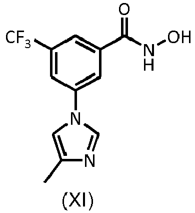 and
reacting the compound of formula (XI) 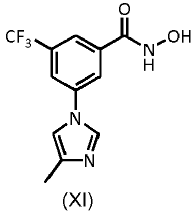 either with a base (B1) or alternatively with a dehydration agent (DA1) in a solvent (S1) to yield the compound of formula (I)

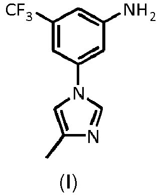

In principle, any strong base (B2) (i.e. a base having a pKb of less than 5) is suitable.Particularly, useful bases (B2) are selected from the group consisting of LiOH, NaOH, KOH, CsOH, Ca(OH)₂, Na₂CO₃, sodium methoxide and K₂CO₃.

The reaction may be carried out in water or in any mixture of water and a solvent (S2) which is freely soluble in water such as C1-C3 linear or branched alkanols, such as methanol, ethanol, isopropanol and tert-butanol.

In another embodiment, the present invention relates to a method for the synthesis of 3-(4-methyl-1H-imidazol-1-yl)-5-(trifluoromethyl)-benzenamine (I),comprising the steps:
reacting a compound of formula (VI) 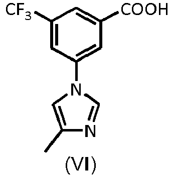
with a C1-C4 linear or branched alkanol in the presence of an acid (A1) to yield the compound of formula (X) 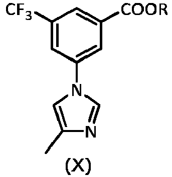
reacting the compound of formula (X) with hydroxylamine or a salt thereof in the presence of a base (B2) in a solvent (S2) to yield the compound of formula (XI) 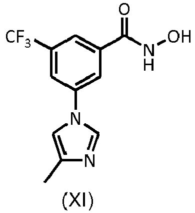 and
reacting the compound of formula (XI) 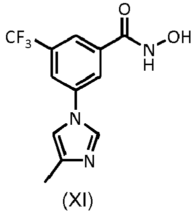
either with a base (B1) or alternatively with a dehydration agent (DA1) in a solvent (S1) to yield the compound of formula (I) 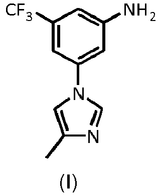
In an embodiment, the acid (A1) has a pKa of less than 5.

In another embodiment, the present invention relates to a method for the synthesis of 3-(4-methyl-1H-imidazol-1-yl)-5-(trifluoromethyl)-benzenamine (I),comprising the steps:
reacting a compound of formula (V) 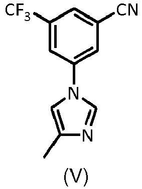
with a base (B3) in the presence of an aqueous mixture comprising water and a solvent (S3) which is freely soluble in water to obtain compound of formula (VI)
reacting a compound of formula (VI) with a C1-C4 linear or branched alkanol in the presence of an acid (A1) to yield the compound of formula (X) 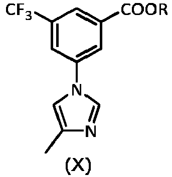
reacting the compound of formula (X) with hydroxylamine or a salt thereof in the presence of a base (B2) in a solvent (S2) to yield the compound of formula (XI) 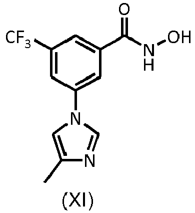 and
reacting the compound of formula (XI) 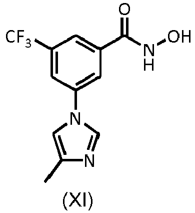
either with a base (B1) or alternatively with a dehydration agent (DA1) in a solvent (S1) to yield the compound of formula (I)

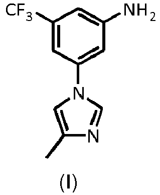

In an embodiment the base (B3) is selected from the group consisting of LiOH, NaOH, KOH, CsOH, Ca(OH)₂, Na₂CO₃ and K₂CO₃.

In an embodiment the solvent (S3) is selected from the group consisting of C1-C3 alkanols, acetone, acetonitrile, glycerol, C2-C4 alkanediols, tetrahydrofurane, dioxane and dimethylsulfoxide.

In a third aspect, the present invention relates to a method for preparing Nilotinib (4-methyl-N-[3-(4-methyl-1H-imidazol-1-yl)-5-(trifluoromethyl)phenyl]-3-[(4-pyridin-3-ylpyrimidin-2-yl)amino)-benzenamine) comprising the following steps:
a) reacting the compound of formula (XI) 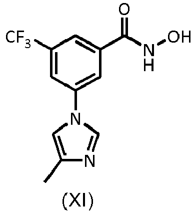 either with a base or alternative with a dehydrating agent in a solvent to yield the compound of formula (I) 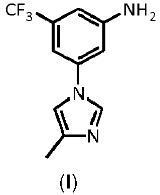
b) reacting the compound of formula (I) with a compound of formula (IIa) optionally in the presence of a dehydrating agent and an inert base and/or suitable catalyst, and optionally in the presence of an inert solvent 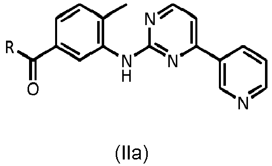 wherein the group COR is selected from the group consisting of carboxylic acids, acyl halides and carboxylic esters;
   to yield nilotinib

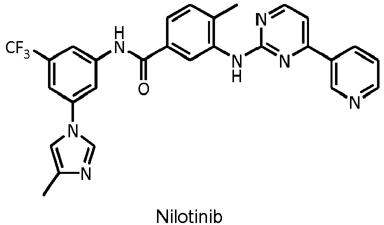

In an embodiment the group COR is a reactive carboxylic ester, i.e.an ester selected from the group consisting of esters of the vinyl type such as actual vinyl esters, carbamoylvinyl esters; alkoxyvinylesters; esters of the amidino type such as N,N'-disubstituted amidino esters and N,N-disubstituted amidino esters; aryl esters such as phenyl esters suitably substitude by electron-withdrawing substituents; cyanomethyl esters; thio esters; amino esters; amido esters or silyl esters. In said embodiment the reaction is preferably carried out using the conditions described in pages 25 to 26 of WO 2004/005281 which are incorporated by reference.

In another embodiment the group COR is an ester derived from a linear or branched C1-C4 alkanol. In said embodiment the reaction is preferably carried out using the conditions described in page 4 of WO 2006/135641 A2 which is incorporated by reference.

In another embodiment the group COR is an acyl halide such as acyl chloride. In said embodiment the reaction is preferably carried out in dichloromethane at reflux temperature.

In a fourth aspect the present invention relates to the use of the compound of formula (XI) in a process for manufacturing Nilotinib (4-methyl-N-[3-(4-methyl-1H-imidazol-1-yl)-5-(trifluoromethyl)phenyl]-3-[(4-pyridin-3-ylpyrimidin-2-yl)amino)-benzenamine).

In an embodiment the process comprises the following steps:
a) reacting the compound of formula (XI) 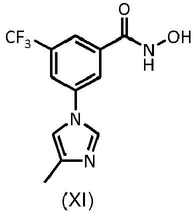 either with a base (B1) or alternatively with a dehydrating agent (DA1) in a solvent (S1) to yield the compound of formula (I) 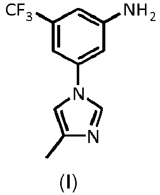
b) reacting the compound of formula (I) with a compound of formula (IIa) optionally in the presence of a dehydrating agent (DA4) and an inert base (B4) and/or suitable catalyst, and optionally in the presence of an inert solvent (S4) 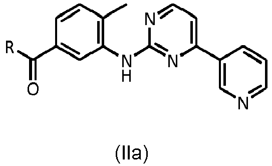 wherein the group COR is selected from the group consisting of carboxylic acids, acyl halides and carboxylic esters, in particular the group COR is selected from the group consisting of vinyl esters; carbamoylvinyl esters; alkoxyvinylesters; N,N'-disubstituted amidino esters; N,N-disubstituted amidino esters; phenyl esters substituted by one to three electron-withdrawing selected from the group consisting of nitro groups, halogens, C1-C4 alkylsulfonyl groups; cyanomethyl esters; thio esters; amino esters; amido esters;silyl esters and carboxylic esters derived from a linear or branched C1-C4 alkanol.

### Examples

The compounds obtained in the examples described below are identified by proton (¹H-NMR) and of carbon-13 (¹³C-NMR) nuclear magnetic resonance data.

The operating frequency and the solvent used for recording the spectrum are indicated in the spectra. The positions of the signals are indicated in δ (ppm) and the signal of the protons of the solvent are used as a reference. The coupling constants (J) are expressed in Hertz. The number of protons of each signal measured by electron integration and the type of signal are indicated in parenthesis, the latter being indicated using the following abbreviations: s (singlet), d (doublet), t (triplet), q (quadruplet), dd (doublet of doublets), ddd (doublet of doublet of doublets), td (triplet of doublets), m (multiplet), br (broad signal).

The following abbreviations are used in the experimental section:

### EXAMPLES

### Comparative Example 1: Preparation of the compound of formula (VII): [3-(4-Methyl-imidazol-1-yl)-5-trifluoromethyl-phenyl]-carbamic acid tert-butyl ester

6.8 g of 3-(4-methyl-imidazol-1-yl)-5-trifluoromethyl-benzoic acid (compound of formula (VI)) and 200 mL of tert-butanol were charged to a 500 mL reactor, then 3.8 g of triethyl amine and 7.6 g of diphenylphosphorylazide were added. The reactor was filled with N₂ and stirred producing a cloudy solution. The mixture was heated to 80 °C in about 30 minutes and the temperature was maintained for 20 hours. The reaction mixture was the cooled to room temperature and the solvent was evaporated off under reduced pressure. The residue was dissolved in 100 ml of ethyl acetate and 100 ml of water. The aqueous phase was extracted with 100 ml of ethyl acetate. The combined organic phases were washed with 100 ml brine and dried over Na₂SO₄. The resulting organic phase was concentrated to afford the crude product which was subsequently purified by column chromatography (silica gel, eluent 2% ethanol in ethyl acetate) to yield 4.5 g (52.3% yield) of a product with a 99% HPLC purity.

### Comparative Example 2: Preparation of the compound of formula (I): 3-(4-Methyl-imidazol-1-yl)-5-trifluoromethyl-benzenamine

3.4 g of compound of formula (VII) [3-(4-Methyl-imidazol-1-yl)-5-trifluoromethyl-phenyl]-carbamic acid tert-butyl ester] and 60 ml of hydrochloric acid in isopropanol were charges to a 250 mL flask. The mixture was heated to 60 °C and the temperature was maintained for 5 hours. The reaction mixture was the cooled to room temperature and concentrated under reduced pressure. The residue was treated with 160 mL of a saturated NaHCO₃ aqueous solution. The resulting solution was extracted with ethyl acetate (160 mL X 3) and the combined organic phase was washed with brine (160 mL X 2), dried over Na₂SO₄ and concentrated to yield the product.
Yield 2.26 g (94.2%); HPLC purity 99%.

### Example 1: Preparation of the compound of formula (VI): 3-(4-methyl-imidazol-1-yl)-5-trifluoromethyl-benzoic acid

30 g of 3-(4-methyl-1H-imidazol-1-yl)-5-(trifluoro-methyl)-benzenamide and 300mL of 1,4-dioxane were charged in a 1L reactor equipped with mechanical stirrer. 322 g of a 7.4% NaOH aq. were added and the mixture was heated to reflux to produce a solution which was maintained at reflux for 12 hours. The solution was then concentrated by vacuum distillation of the solvent up to half the initial volume. The reaction mixture was cooled to room temperature.

pH was adjusted to a value of 4.0-5.0 by addition of 18% hydrochloric acid maintaining the temperature below 25 °C.

The reaction mixture was cooled to 10°C and maintain at this temperature while stirring for 1 hour.

The precipitate was filtered under vacuum, and the cake was washed with 60 ml of water twice. The resulting solid was dried under vacuum at 50°C.

Yield 28.5 g (88%); HPLC purity 98.8%; off-white solid.

### Example 2: Preparation of the compound of formula (X-a): 3-(4-methyl-imidazol-1-yl)-5-trifluoromethyl-benzoic acid, methyl ester

50 g of 3-(4-methyl-imidazol-1-yl)-5-trifluoromethyl-benzoic and 400 mL methanol were charged to a 1L reactor. 37 g of 98% H₂SO₄ were added dropwise into the solution. The reaction mixture was heated to reflux. The reaction was maintained at reflux for 24 hours and the concentrated to dryness under reduced pressure. The residue was dissolved in 500 ml of ethyl acetate (500 mL). The resulting solution was treated by adding 20 % Na₂CO₃ aqueous solution until a pH value of 9-10 was obtained. The organic phase was decanted and the aqueous phase was extracted with ethyl acetate (2 X 250 ml). The combined organic phase was washed with brine and dried over Na₂SO₄. The resulting solution was concentrated to dryness under reduced pressure to yield 3-(4-methyl-imidazol-1-yl)-5-trifluoro-methyl-benzoic acid, methyl ester.
Yield: 47 g (89.5%); HPLC purity: 99.4%; off-white solid.

### Example 3: Preparation of the compound of formula (XI): N-hydroxy-3-(4-methyl-imidazol-1-yl)-5-trifluoromethyl-benzamide

34,40 g of 3-(4-methyl-imidazol-1-yl)-5-trifluoro-methyl-benzoic acid, methyl ester, 17.16 g of HONH₂·HCl and 344 ml of methanol were charged to a 1 L jacketed reactor. The mixture was stirring at 25 °C for 10 minutes. Then, 114.44 g of a 20 % NaOMe solution in methanol was added dropwise during 30 min. The resulting mixture was heated to reflux and maintained at reflux for 1 hour. The reaction mixture was then cooled to 25 °C. The pH of the mixture was adjusted to a value of 8.5 by addition of a 3.6 % HCl aqueous solution. The resulting solution was concentrated under vacuum up to 2/3 the initial volume (220g fraction) and then cooled to 0 °C in 30 min. The mixture was maintained at 0 °C for 1 hour. Then, the mixture was filtered and the cake was washed with water (60 mLX2). The solid was dried over vaccum at 55 °C.

Yield: 27.2 g (78.8%); HPLC purity: 99.5% (HPLC ); off-white solid.

### Example 4: Preparation of the compound of formula (I): 3-(4-Methyl-imidazol-1-yl)-5-trifluoromethyl-benzenamine

27.0 g of N-hydroxy-3-(4-methyl-imidazol-1-yl)-5-trifluo-romethylbenzamide, 10.2 g of Na₂CO₃ and 135 ml of DMSO were charge to a 1 L flash. The mixture was heated to 140 °C and maintained at this temperature for 6 hours. The mixture was then cooled to 25°C. 405.0 ml of water were added dropwise and the resulting mixture was cooled to 10°C and maintained at this temperature while stirring for 3 hours. A precipate developed which was filtered under vacuum. The cake was washed with 56 ml of water twice and the resulting solid dried under vacuum at 55°C.
Yield 19.2 g (84.1%); HPLC purity 98.5% (HPLC); off-white solid.

### Example 5: Preparation of the compound of formula (I) : 3-(4-Methyl-imidazol-1-yl)-5-trifluoromethyl-benzenamine

2 g of N-hydroxy-3-(4-methyl-imidazol-1-yl)-5-trifluo-romethylbenzamide, 1.74 g CDI and 20 mL CH₃CN were charged to a 100 mL reactor. The mixture was heated to 40 C until a clear green solution was obtained. The mixture was maintained at 40 °C for 0.5 hour and a brown solution was obtained. 5 mL of MeOH were added into the reaction. The resulting solution was heated to reflux and the reflux was maintained for 4 hours. 26.5 g of a 7.4% NaOH aqueous solution was added into the reaction mixture which was maintained at reflux for 1 hour. Then, the mixture was concentrated to the half of initial volume under reduced pressure and filtered. The resulting cake was washed with water (5 mLX2) and dried over vaccum at 50 °C
Yield: 88.8%; HPLC purity: 87%; white solid.

The examples 1 to 5 show that the process of the invention for obtaining compound of formula (I) starting from compound of formula (V) affords the desired compound with an overall yield higher than 45%.

## Claims

1. A method for preparing 3-(4-methyl-1H-imidazol-1-yl)-5-(trifluoromethyl)-benzenamine (I) by reacting the compound of formula (XI) 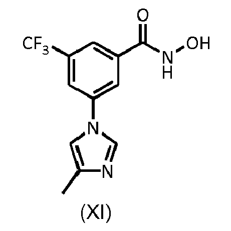 either with a base (B1) or alternatively with a dehydration agent (DA1) in a solvent (S1) to yield the compound of formula (I)

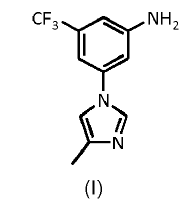

2. A method according to claim 1 wherein the base (B1) has a pKb of less than 5.

3. A method according to anyone of claims 1 and 2 wherein the reaction is carried out in the presence of a base (B1) selected from the group consisting of inorganic hydroxides and carbonates.

4. A method according to claim 1 wherein the reaction is carried out in the presence of a dehydrating agent (DA1) selected from the group consisting of dicyclohexylcarbodiimide, carbonyldiimidazole, thionyl chloride, acetic anhydride, p-toluenesulfonyl chloride, cyanuric chloride, phosphorous pentoxide and N-benzyl-N-(3-dimethylaminopropyl)carbodiimide.

5. A method according to anyone of claims 1 to 4 further comprising the previous step of reacting the compound of formula (X) 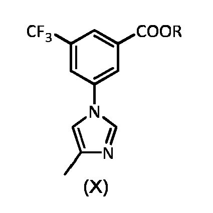 with hydroxylamine or a salt thereof in the presence of a base (B2) in a solvent (S2) to yield the compound of formula (XI)

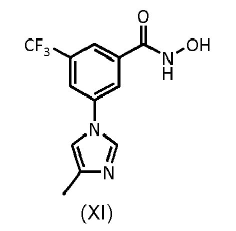

6. A method according to claim 5 wherein the base (B2) has a pKb of less than 5.

7. A method according to any one of claims 5 to 6 wherein the solvent (S2) is selected from the group consisting of C1-C4 linear or branched alkanols.

8. A method according to anyone of claim 5 to 7 further comprising the previous step of reacting a compound of formula (VI) 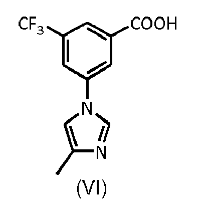 with a C1-C4 linear or branched alkanol in the presence of an acid (A1) to yield the compound of formula (X)

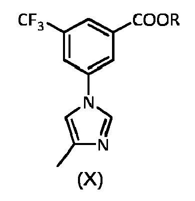

9. A method according to claim 8 wherein the acid (A1) has a pKa of less than 5.

10. A method according to anyone of claims 8 to 9 further comprising the previous step of reacting a compound of formula (V) 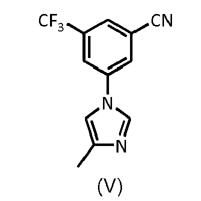 with a base (B3) in the presence of an aqueous mixture comprising water and a solvent (S3) which is freely soluble in water to obtain compound of formula (VI)

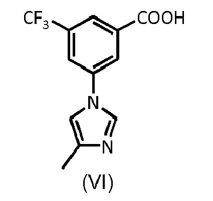

11. A method according to claim 10 wherein the base (B3) is selected from the group consisting of LiOH, NaOH, KOH, CsOH, Ca(OH)₂, Na₂CO₃ and K₂CO₃.

12. A method according to anyone of claims 10 to 11 wherein the solvent (S3) is selected from the group consisting of C1-C3 alkanols, acetone, acetonitrile, glycerol, C2-C4 alkanediols, tetrahydrofurane, dioxane and dimethylsulfoxide.

13. A compound of formula (XI)

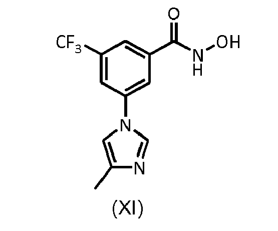

14. Use of the compound of formula (XI) in a process for manufacturing Nilotinib (4-methyl-N-[3-(4-methyl-1H-imidazol-1-yl)-5-(trifluoromethyl)phenyl]-3-[(4-pyridin-3-ylpyrimidin-2-yl)amino)-benzenamine).

15. Use according to claim 14 wherein the process comprises the following steps:
c) reacting the compound of formula (XI) 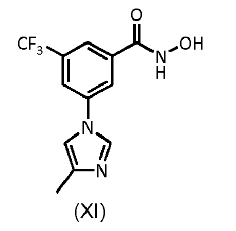 either with a base (B1) or alternatively with a dehydrating agent (DA1) in a solvent (S1) to yield the compound of formula (I) 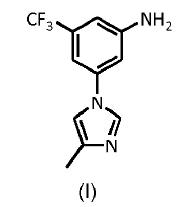
d) reacting the compound of formula (I) with a compound of formula (IIa) optionally in the presence of a dehydrating agent (DA4) and an inert base (B4) and/or suitable catalyst, and optionally in the presence of an inert solvent (S4) 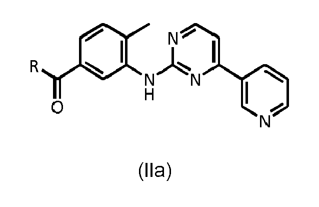 wherein the group COR is selected from the group consisting of carboxylic acids, acyl halides and carboxylic esters.
